# EUROPEAN PATENT SPECIFICATION

(11) **EP 1 014 891 B1**
(45) Date of publication and mention of the grant of the patent: **02.06.2004**
(21) Application number: 99938394.6
(22) Date of filing: 16.07.1999
(51) Int. Cl.: A61F 2/06

(54) **EXPANDABLE STENT**
EXPANDIERBARER STENT
TUTEUR EXTENSIBLE

(30) Priority: 17.07.1998 FR 9809137; 30.03.1999 FR 9903962
(43) Date of publication of application: 05.07.2000
(73) Proprietor: B. BRAUN MELSUNGEN AG, 34212 Melsungen (DE)
(72) Inventor: ROUSSIGNE, Maurice, F-86000 Poitiers (FR); NADAL, Guy, F-86000 Poitiers (FR)
(74) Representative: Lerner, François
(86) International application number: PCT/EP1999/006108
(87) International publication number: WO 2000/003661

(56) References cited:
- EP-A- 0 806 190
- WO-A-98/22159
- WO-A-98/40035
- DE-U- 29 702 671
- DE-U- 29 708 689
- DE-U- 29 708 879
- US-A- 5 911 754

## Description

The invention relates to an expandable support for an anatomical lumen (or duct), and in particular for a blood vessel.

Such a support is typically called a stent when it is a duct expander that is to support the wall of the duct in order to prevent the passage there from being (too) narrow. Expandable supports are known from the documents EP-A-0 806 190, WO-A-9 822 159, DE-U-29 708 879 and DE-U-29 702 671.

The object of the invention is to improve the efficiency and the tolerance by patients of existing supports. The flexibility and the suppleness of the support have been particularly researched in order to promote its implantation and the following of the natural bends in the duct concerned, in particular if it is a coronary vessel.

With regard to implantation, particular attention has also been paid to the ability of the support to be implanted endoluminally (SELDINGER method, in particular) by means of an insertion device having a small diameter.

To that end, an important feature of the invention provides that the proposed expandable support (or stent) has a unitary structure and a principal axis and a succession of axially aligned tubular stages, at least some of those stages being defined by a regular pattern having consecutive loops closed on themselves, with a succession of hairpin-shaped apices which are linked consecutively in pairs by a branch, each branch having two inflexion points defining a double inflexion region, the loops being arranged in phase from one stage to an adjacent stage and being connected to one another by small connecting bars linking the apex region of a loop of a given stage to the region of the inflexion points of a loop corresponding to it axially, in a preceding or following adjacent stage, in an alternating manner on the perimeter of the "given stage" concerned.

Still for the purpose of adapting the flexibility to the bends in the duct, especially if it is a coronary duct, while nevertheless ensuring efficient internal support of the duct, a supplementary feature of the invention recommends that the two inflexion points of each branch define substantially an open "S" so that the branch does not have a curve summit.

Likewise in order to improve the balance between flexibility and resistance to crushing, it is recommendable for the connecting bars to be substantially in a zigzag arrangement, from stage to stage.

Still with the same aim, and also with the aim of ensuring an appropriate size for the cells (defined by the loops and bars), another feature of the invention recommends that the loops of two adjacent stages partially overlap one another.

And, in order to promote a balanced opening of the stent from stage to stage along its axis, yet another feature recommends that, in a given stage, the connecting bars between stages connect the loops of the structure alternately to a preceding adjacent stage or to a following adjacent stage, starting from the linking branch arranged alternately on one or other side of the central apex of the consecutive loops.

It should also be noted that an important feature of the invention additionally recommends:
- that the double inflexion region be arranged at the median portion of the branch under consideration,
- that, on each side of that region, the branch have a portion, for connection to the corresponding apex, which extends substantially parallel to the axis of the stent, in a substantially rectilinear manner,
- that the apices be rounded,
- and that each connecting bar be substantially rectilinear and extend parallel to the axis of the stent in order to connect two branches arranged (substantially) in the axial extension one of the other, in two adjacent stages.

Thus, the design of the stent structure is prevented from being overcomplicated while obtaining appropriate dimensions for the cells and a structure "design" conferring on the structure an optimised relationship between flexibility and crushing resistance.

The bearing and the positioning conditions of the support, once it is implanted (and therefore radially deployed) in the duct have also been researched because the support must have, at its free ends, a diameter in its deployed state at least equal to that of the remainder of the structure and must be able to stay substantially in its implantation position without necessarily comprising hooks or other means for securing to the duct wall.

It is therefore by relative friction and by its radial bearing force (which is opposed by the radial resistance of the duct wall) that the support holds itself in position.

The ability of the support to follow the natural bends in the duct concerned has also been taken into consideration.

In order to satisfy those requirements, a solution suggested by the invention consists in particular in proposing a support, the above-mentioned the coaxial stages of which define a substantially cylindrical wall surface having a circular cross-section, the support being characterised in that at least one of said stages, respectively the first and the last, arranged at its proximal and distal free ends, comprises free-end apices having a widened portion along the circumferential wall surface, with at least one component along the perimeter of the structure, in a plane perpendicular to the axis of said structure.

With apices thus widened at the proximal and/or distal free ends of its structure, the support provides a widened surface for bearing against the inner wall of the duct, thus permitting the creation at those end sites of regions of increased resistance to any forces which would tend to displace the support along the duct.

In that connection, it has also been noted that it is preferably exclusively along the perimeter of the proximal and/or distal free ends that it is advantageous to promote (that is to say, increase) the contact area between the support and its receiving duct.

Thus, another feature of the invention provides that the widened portion of the above-mentioned free-end apices is terminated, parallel to the axis of the structure, by a rectilinear portion in a plane substantially perpendicular to said axis.

It will be appreciated that the presence of such a "rectilinear portion" also avoids the presence at that site of a shape which could be aggressive with respect to the duct wall.

Also in connection with that same consideration, another feature of the invention recommends that the above-mentioned widened apex portions each have a substantially triangular shape with rounded angles.

It will be appreciated that such a triangular shape enables a lengthening of the contact surface between the support and the inner wall facing the duct to be reconciled with the constraint of a shape which promotes that contact, in particular along the perimeter of the end stages concerned, without leading to a shape which could traumatise the duct wall.

A more detailed description of the invention will now be provided with reference to the appended drawings in which:
- Figure 1 is a general front view of an expandable support according to the invention,
- Figure 2 is an enlarged view of the detail marked II in Figure 1
   - Figure 3 shows a "basic" loop (enlarged detail III of Figure 1, without the bar),
   - Figure 4 is a general view of another first embodiment of an expandable support according to the invention;
   - Figure 5 is an enlarged local view of the detail marked V in Figure 4;

The structures shown in the enclosed Figures are vessel expanders (in particular coronary vessel expanders), typically called stents.

The form of their fundamental structure as illustrated could, however, be used in other types of anatomical support, such as, for example, prostheses for aneurysm (production of the stent associated with a supple sleeve which channels the blood), or an internal support for a portion of the oesophagus or for a portion of the urethra, for example.

The expander 1 represented is in particular usable in the treatment of a stenosis.

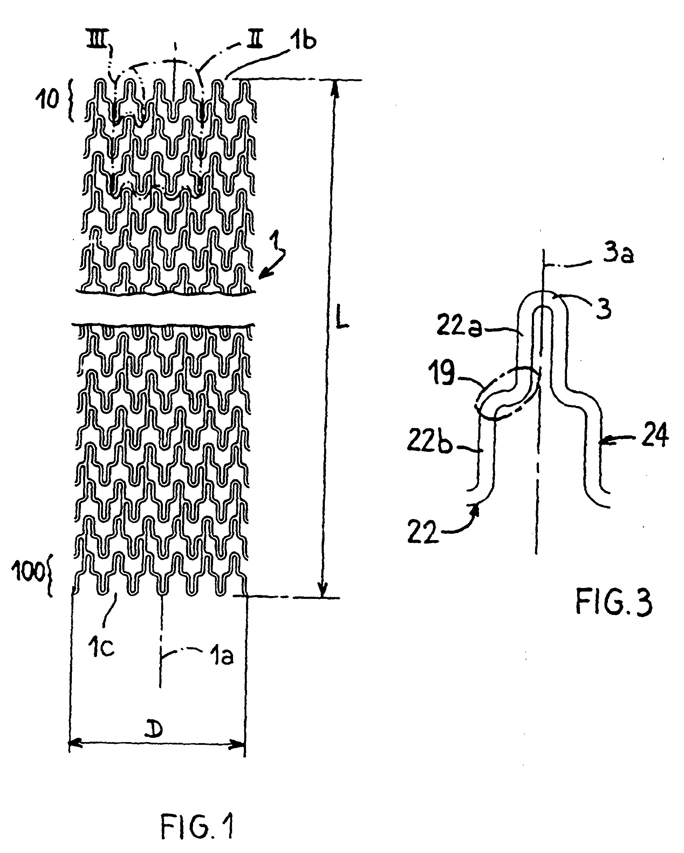

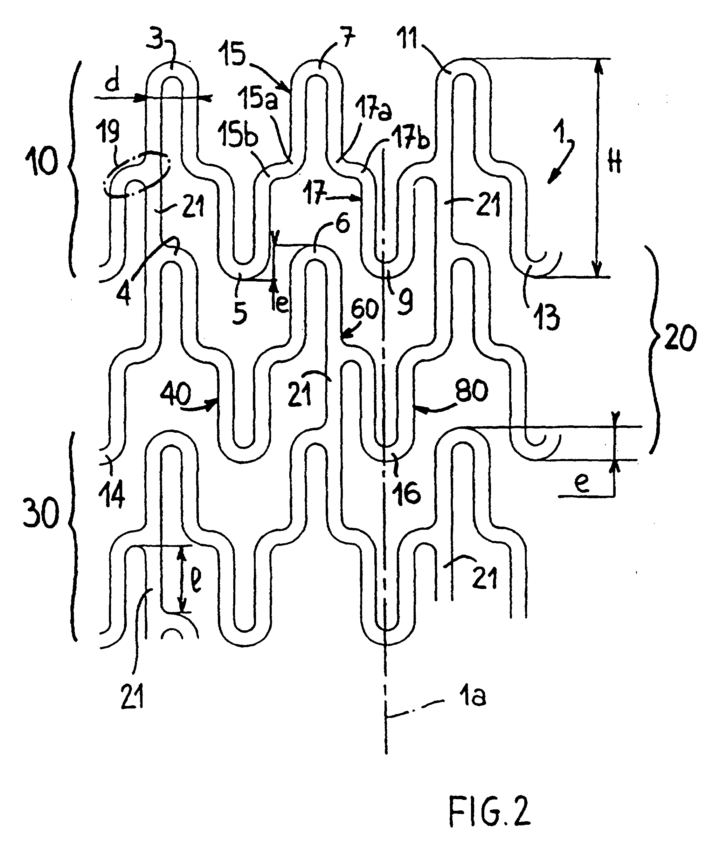

The expander is a hollow cylindrical tube, of axis 1a, which may occupy, in particular, two states: a radially confined state as in Figures 1 and 2, and a radially deployed state, which has not been shown.

Typically, its axial length is from 9 mm to 30 mm, while its diameter (in the confined state) may be of the order of from 1.3 to 3 mm, which diameter can increase to 4 or even 6 mm in the radially deployed state.

The support 1 may in particular be manufactured by laser cutting from a thin plate of metal, such as type 316L stainless steel, of the order of one tenth of a millimetre thick.

Manufacture from a material "having heat shape memory", such as "nitinol" (registered trademark) would also be possible.

The result is an open integral structure (in a single piece).

Figure 2 shows more clearly the "design" of the structure which, in this particular case, is identical over the entire surface thereof, except for the proximal 1b and distal 1c free ends where, respectively, the first and last stages 10, 100, are found.

Thus, the structure may be described as having a succession of stages, such as those marked 10, 20, 30 in Figure 2, which are all spaced along the axis 1a to form individually an annular cylinder of an individual height of from 1 mm to 1.5 mm.

In this particular case, the diameter of the ring defined at each stage is constant over the entire length of the stent, both in the confined state and in the radially deployed state.

It may be considered that each stage extends along a cylinder portion of axis 1a and that, except for the end stages 10, 100, each of the intermediate stages located between them is defined by a regular pattern having consecutive loops closed on themselves with a succession of apices marked 3, 5, 7, 9, 11, 13, in the case of some of them, in Figure 2.

All those apices located at the axial ends (that is to say, terminating the loops of the stage under consideration along an axis parallel to the axis 1a) will be regarded as being "hairpin-shaped" although each apex is rounded in order to be atraumatic.

In one and the same stage, two successive apices are connected by a linking branch (such as 15 and 17, respectively, for the apices 5-7 and 7-9 of the stage 10, in Figure 2).

Each branch has two inflexion points, such as 15a, 15b and 17a, 17b, which define a double inflexion region, to the extent that they are relatively close to one another, on the branch concerned.

The thus defined loops of the stages are arranged opposite one another from one stage to another, including as regards the first and last stages 10, 100.

Figure 3 shows the design of a "basic" loop of the structure.

It will thus be appreciated that each basic loop has a central peak apex, such as that marked 3 in Figure 3, connected to two linking branches 22,24 which have a double inflexion region and which are arranged one on each side of the axis of symmetry 3a. Each branch therefore comprises two portions (such as 22a and 22b in the case of the branch 22). Each portion is preferably (substantially) parallel to the axis 1a (or 3a).

Advantageously, each double inflexion region, such as 19 in the case of the branch 22, is arranged at the median portion of the branch (approximately at mid-height) and the two inflexion points are close to one another, in such a manner as to define a (short) shoulder which widens the loop at its base until it reaches approximately three times the width (d) of its peak, at the site of its central apex (Figure 2).

The double inflexion region preferably has the shape of an open "S", that is to say, a concave curve followed by a convex curve, without an intermediate curve summit, thus preventing excessive tortuousness.

Two successive stages (such as 20 and 30) where the loops are arranged in phase, are connected to one another by small connecting bars 21.

In the Figures, the bars are in a zigzag arrangement, from stage to stage, that is to say that, considering a loop (such as 60) of a given stage, which loop is connected, parallel to the axis 1a, by a bar 21 to the loop corresponding to it in the following adjacent stage 30, the two loops 40, 80 bordering it on each side are each connected by another bar 21 to the loop arranged in their axial continuation, in the preceding stage 10.

With the exception of the two end stages 10, 100 (where the loops are connected only on one side, to a single stage) the loops of all the intermediate stages are therefore connected, per stage, on the perimeter of the structure, alternately to the loop located in their axial extension in the preceding stage or in the following stage.

The "hairpin-shaped" form of the region of the apices 3, 4, 5, 6, 7, ..., will also have been noted, although each apex is rounded in order to be atraumatic.

Bearing in mind the above, and in particular the arrangement, without a phase shift, of the loops from one stage to an adjacent stage, the connecting bars 21 may be rectilinear bars which each extend parallel to the axis 1a in order to connect the double inflexion region, such as 19, to the apex region (such as 4) of the loop (such as 40) facing it axially. Other forms ("C"-shaped, "<"-shaped, ...) could be provided for. Preferably, however, all the bars will be in the same form.

In the embodiment illustrated, it will be appreciated that, in order to balance the structure, the bars 21 of the loops 40 and 60 (this is reproducible over the remainder of the structure) connect the loops under consideration alternately to a "preceding" adjacent stage 10 or to a "following" adjacent stage 30, starting from the linking branch arranged alternately on one and the other side of the axial-end apex considered (such as 4,14, 6, 16, ...) (see connection at the top to the left of the apex 4 of the loop 40, then at mid-height to the right of the apex 6, at the site of the double inflexion of the loop 60 between the axial-end apices 6 and 16).

The axial length of the bars 21 is also advantageously such that the loops of the two adjacent stages partially overlap axially, preferably over approximately from one to a few tenths of a millimetre (the thickness of the filament of material forming the loops), see height e in Figure 2.

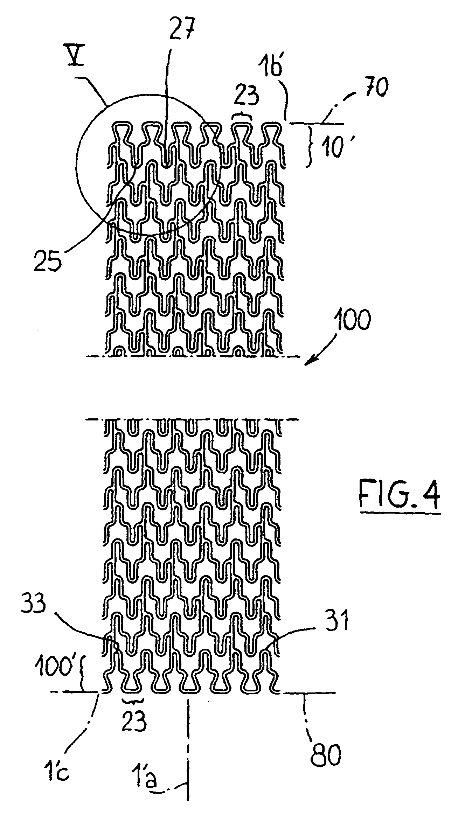

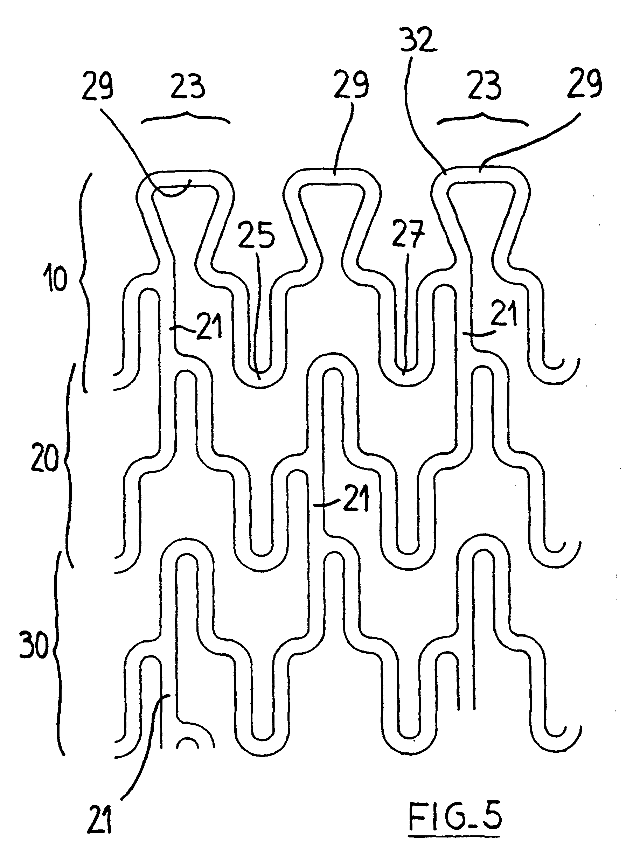

In the embodiment of Figures 4 and 5,each apex of the loops constituting the first and last stages 10', 100' has, at the site of the perimeter of the corresponding free ends 1'b, 1'c, a widened portion 23 which is found nowhere else and, in particular, is not found at the site of the "intermediate" apices, such as those marked 25, 27, 31, 33 in Figure 4 or 5 (substantially "hairpin-shaped" apex).

In order to promote the bearing and retaining effect sought in relation to the anatomical duct, each widened portion 23 extends along the circumferential wall surface of the structure 100 considered as a whole, that is to say, the widened portions do not project radially, either towards the inside or towards the outside of the general cylindrical surface of the structure.

In the case in point, each widened portion 23 incorporates a rectilinear "peak" portion 29 (which defines its widened apex region) in a plane substantially perpendicular to the axis 1'a (plane marked by the line 70 in the case of the free end 1'b and by the line 80 in the case of the distal end 1'c, in Figure 4).

Bearing in mind the general form adopted for the loops of the structure, Figures 4 and 5 also clearly show that each widened portion of the end apices 23 preferably has a substantially "triangular" shape with rounded angles 32 which is particularly adapted to the "design" as a whole already presented.

As in the case of Figures 1 to 3, the support of Figures 4 and 5 is provided to be radially deployed starting from the form illustrated in the Figures starting from their illustrated radially confined state, under the effect in particular of an inflatable internal balloon (not shown), or even a thermal effect if the material used is a "material having heat shape memory".

It will be further noted that each stage of the above-mentioned structures develops, while closing on itself (in the manner of a basic ring) in a plane perpendicular to the longitudinal axis of the structure, in such a manner that the structure may be regarded as having a succession of annular stages regularly spaced along said axis. In addition, each stage is defined by a fine strip of looped material having a succession of troughs and peaks.

Further, the use of the above supports 1, 100 is conventional.

After placing them around their inflatable balloon (if they do not have a heat shape memory), they are placed in an insertion device and they are introduced into their (coronary) receiving duct inside which they are deployed by inflating the balloon until they bear against the inner wall of the duct (vessel). The introduction system is then withdrawn and the support is left in place.

## Claims

1. Expandable support for an anatomical duct, the support having a unitary structure and having a principal axis (1a) and a succession of axially aligned tubular stages (10, 20, 30), at least some of those stages being defined by a regular pattern having consecutive loops (40, 60) closed on themselves, with a succession of hairpin-shaped apices (3, 4, 5, 6) which are linked consecutively in pairs by a branch (15, 17), each branch having two inflexion points (15a, 15b) defining a double inflexion region (19), the loops being arranged in phase from one stage to an adjacent stage and being connected to one another by small connecting bars (21) linking the apex region of a loop of a given stage to the region (19) of the inflexion points of a loop corresponding to it axially, in a preceding or following adjacent stage, in an alternating manner on the perimeter of said stage.

2. Support according to claim 1, **characterised in that**:
- the double inflexion region (19) is arranged at the median portion of the branch under consideration,
- on each side of that region, the branch has a portion (22a, 22b), for connection to the corresponding apex, which extends substantially parallel to the axis of the support and which is substantially rectilinear,
- the apices (3, 4) are rounded,
- and each connecting bar (21) is substantially rectilinear and extends parallel to the axis (1a) of the support in order to connect two branches arranged in the axial extension one of the other, in two adjacent stages.

3. Support according to claim 1 or claim 2, **characterised in that** the two inflexion points of each branch define substantially an open "S", so that the branch does not have a curve summit.

4. Support according to any one of the preceding claims, **characterised in that** the connecting bars (21) are substantially in a zigzag arrangement, from stage to stage.

5. Support according to any one of the preceding claims, **characterised in that** the loops of two adjacent stages (10, 20, 30) partially overlap one another.

6. Support according to any one of the preceding claims, **characterised in that**, in a given stage, the bars (21) connect the loops alternately to a preceding adjacent stage or to a following adjacent stage, starting from the linking branch arranged alternately on one or other side of the central apex (3, 4, 6, 7, 8, 11) of the consecutive loops.

7. Support according to anyone of the preceding claims, **characterised in that** :
- the support has along its axis a proximal free end and a distal free end,
- the support also has a succession of tubular stages (10, 20),
- the coaxial stages of the support define a substantially cylindrical wall surface having a circular cross-section, and
- at least one of said stages, respectively the first (10, 10') and the last (100, 100') arranged at the proximal and distal free ends, comprises free-end apices having a widened portion (23) along the circumferential wall surface, with at least one component along the perimeter.

8. Support according to claim 7, **characterised in that**, between two successive free-end apices (23) of the first (10') and/or of the last (100') stages of the structure, the corresponding loop has at least one hairpin-shaped apex (25, 27).

9. Support according to claim 7 or claim 8, **characterised in that** the widened portion of the free-end apices is terminated, parallel to the axis of the structure, by a rectilinear portion (29) in a plane (70, 80) which is substantially perpendicular to said axis.

10. Support according to claims 7 to 9, **characterised in that** the widened portion of the free-end apices has a substantially triangular shape having rounded angles (32).

## Patentansprüche

1. Expandierbare Stütze für einen anatomischen Gang, wobei die Stütze einen Einheitsaufbau, eine Hauptachse (1a) und eine Folge von axial in Flucht liegenden rohrförmigen Stufen (10, 20, 30) hat, wobei mindestens einige dieser Stufen durch ein regelmäßiges Muster mit aufeinanderfolgenden Schlingen (40, 60) gebildet sind, die auf sich selbst geschlossen sind, mit einer Folge von haarnadefförmigen Scheiteln (3, 4, 5, 6), die aufeinanderfolgend paarweise durch eine Abzweigung (15, 17) verbunden sind, wobei jede Abzweigung zwei Biegepunkte (15a, 15b) hat, welche einen Doppelbiegebereich (19) bilden, die Schlingen von einer Stufe zu einer benachbarten Stufe in Phase angeordnet und durch kleine Verbindungsstäbe (21) miteinander verbunden sind, welche den Scheitelbereich einer Schlinge einer gegebenen Stufe mit dem Bereich (19) der Biegepunkte einer diesem entsprechenden Schleife in einer vorhergehenden oder folgenden benachbarten Stufe abwechselnd auf dem Umfang der Stufe axial verbinden.

2. Stütze nach Anspruch 1, **dadurch gekennzeichnet, daß**:
- der Doppelbiegebereich (19) an dem mittleren Abschnitt der betreffenden Abzweigung angeordnet ist, auf jeder Seite dieses Bereiches die Abzweigung einen Abschnitt (22a, 22b) für die Verbindung des entsprechenden Scheitels hat, wobei sich der Abschnitt im wesentlichen parallel zu der Achse der Stütze erstreckt und im wesentlichen geradlinig ist,
- die Scheitel (3, 4) gerundet sind und
- jeder Verbindungsstab (21) im wesentlichen gerade ist und sich parallel zu der Achse (1a) der Stütze erstreckt, um zwei Abzweigungen, die in der axialen Verlängerung zueinander angeordnet sind, in zwei benachbarten Stufen zu verbinden.

3. Stütze nach Anspruch 1 oder Anspruch 2, **dadurch gekennzeichnet, daß** die Doppelbiegepunkte jeder Abzweigung ein im wesentlichen offenes "S" bilden, so daß die Abzweigung nicht einen Kurvenscheitelpunkt hat.

4. Stütze nach einem der vorhergehenden Ansprüche, **dadurch gekennzeichnet, daß** die Verbindungsstäbe (21) sich von Stufe zu Stufe in einer im wesentlichen Zickzack-Anordnung befinden.

5. Stütze nach einem der vorhergehenden Ansprüche, **dadurch gekennzeichnet, daß** die Schlingen zweier benachbarter Stufen (10, 20, 30) sich einander teilweise überlappen.

6. Stütze nach einem der vorhergehenden Ansprüche, **dadurch gekennzeichnet, daß** in einer gegebenen Stufe die Stäbe (21) die Schlingen abwechselnd mit einer vorhergehenden benachbarten Stufe oder einer folgenden benachbarten Stufe unter Beginnen von demjenigen Verbindungszweig, der abwechselnd auf der einen oder der anderen Seite des mittleren Scheitels (3, 4, 6, 7, 8, 11) der aufeinanderfolgenden Schlingen angeordnet ist, verbinden.

7. Stütze nach einem der vorhergehenden Ansprüche, **dadurch gekennzeichnet, daß**:
- die Stütze entlang ihrer Achse ein proximales freies Ende und ein distales freies Ende hat,
- die Stütze auch eine Folge von rohrförmigen Stufen (10, 20) hat,
- die koaxialen Stufen der Stütze eine im wesentlichen zylindrische Wandoberfläche mit einem Kreisquerschnitt bilden und
- mindestens eine der Stufen, die erste (10, 10') bzw. die letzte (100, 100'), die an dem proximalen bzw. distalen freien Ende angeordnet sind, Scheitel mit freiem Ende aufweist mit einem verbreiterten Abschnitt (23) längs der Umfangswandoberfläche, wobei mindestens eine Komponente längs des Umfanges liegt.

8. Stütze nach Anspruch 7, **dadurch gekennzeichnet, daß** zwischen zwei aufeinanderfolgenden Scheiteln (23) mit freiem Ende der ersten (10') undioder der letzten (100') Stufe des Aufbaues die entsprechende Schlinge mindestens einen haarnadelförmigen Scheitel (25, 27) hat.

9. Stütze nach Anspruch 7 oder Anspruch 8, **dadurch gekennzeichnet, daß** der verbreiterte Abschnitt der Scheitel mit freiem Ende parallel zu der Achse des Aufbaues durch einen geradlinigen Abschnitt (29) in einer Ebene (70, 80) begrenzt ist, die zu der Achse im wesentlichen senkrecht liegt.

10. Stütze nach den Ansprüchen 7 bis 9, **dadurch gekennzeichnet, daß** der verbreiterte Abschnitt der Scheitel mit freiem Ende eine im wesentlichen dreieckige Gestalt mit gerundeten Winkeln (32) hat.

## Revendications

1. Support expansible pour conduit anatomique, le support ayant une structure unitaire et possédant un axe principal (1a) et une série d'étages tubulaires alignés axialement (10, 20, 30), certains au moins de ces étages étant délimités par un motif régulier ayant des boucles consécutives (40, 60) fermées sur elles-mêmes, avec une série de sommets (3, 4, 5, 6) en épingle à cheveux qui sont reliés consécutivement par paires par une dérivation (15, 17), chaque dérivation ayant deux points d'inflexion (15a, 15b) qui délimitent une région d'inflexion double (19), les boucles étant disposées en phase d'un étage à un étage adjacent et étant raccordées les unes aux autres par de petites barres (21) de raccordement qui relient la région de sommet d'une boucle d'un étage donné à la région (19) des points d'inflexion d'une boucle qui lui correspond axialement, dans un étage adjacent précédent ou suivant, en alternance à la périphérie de l'étage.

2. Support selon la revendication 1, **caractérisé en ce que** :
- la région d'inflexion double (19) est disposée dans la partie médiane de la dérivation considérée,
- de chaque côté de cette région, la dérivation a une partie (22a, 22b) destinée à être raccordée au sommet correspondant, qui s'étend en direction pratiquement parallèle à l'axe du support et qui est pratiquement rectiligne,
- les sommets (3, 4) sont arrondis, et
- chaque barre de raccordement (21) est pratiquement rectiligne et s'étend parallèlement à l'axe (1a) du support afin qu'elle raccorde deux dérivations placées dans le prolongement axial l'une de l'autre, dans deux étages adjacents.

3. Support selon la revendication 1 ou 2, **caractérisé en ce que** les deux points d'inflexion de chaque dérivation délimitent pratiquement un "S" ouvert tel que la dérivation n'a pas une crête de courbe.

4. Support selon l'une quelconque des revendications précédentes, **caractérisé en ce que** les barres de raccordement (21) ont une disposition pratiquement en zigzags d'un étage à un autre.

5. Support selon l'une quelconque des revendications précédentes, **caractérisé en ce que** les boucles de deux étages adjacents (10, 20, 30) se recouvrent partiellement.

6. Support selon l'une quelconque des revendications précédentes, **caractérisé en ce que**, dans un étage déterminé, les barres (21) raccordent les boucles en alternance avec un étage adjacent précédent ou un étage adjacent suivant, en commençant à la dérivation de liaison disposée en alternance d'un côté ou de l'autre du sommet central (3, 4, 6, 7, 8, 11) des boucles consécutives

7. Support selon l'une quelconque des revendications précédentes, **caractérisé en ce que** :
- le support possède, le long de son axe longitudinal, une extrémité proximale libre et une extrémité distale libre,
- le support possède aussi une série d'étages tubulaires (10, 20),
- les étages coaxiaux du support délimitent une surface de paroi pratiquement cylindrique ayant une section circulaire, et
- l'un au moins des étages, respectivement le premier (10, 10') et le dernier (100, 100') disposés aux extrémités libres proximale et distale, comprend des sommets d'extrémité libre ayant une partie élargie (23) le long de la surface de paroi circonférentielle, avec au moins un élément suivant la périphérie

8. Support selon la revendication 7, **caractérisé en ce que**, entre deux sommets successifs (23) d'extrémité libre des premier (10') et/ou dernier (100') étages de la structure, la boucle correspondante a au moins un sommet (25, 27) en épingle à cheveux.

9. Support selon la revendication 7 ou 8, **caractérisé en ce que** la partie élargie des sommets d'extrémité libre est terminée, parallèlement à l'axe de la structure, par une partie rectiligne (29) dans un plan (70, 80) qui est pratiquement perpendiculaire à l'axe.

10. Support selon les revendications 7 à 9, **caractérisé en ce que** la partie élargie des sommets d'extrémité libre a une forme pratiquement triangulaire ayant des angles arrondis (32).
